# EUROPEAN PATENT SPECIFICATION

(11) **EP 3 897 641 B1**
(45) Date of publication and mention of the grant of the patent: **15.11.2023**
(21) Application number: 19828759.1
(22) Date of filing: 20.12.2019
(51) Int. Cl.: A61K 31/496, A61P 25/16

(54) **TREATMENT OF MOVEMENT DISORDERS**
BEHANDLUNG VON BEWEGUNGSSTÖRUNGEN
TRAITEMENT DE TROUBLES DU MOUVEMENT

(30) Priority: 20.12.2018 EP 18214575
(43) Date of publication of application: 27.10.2021
(73) Proprietor: Contera Pharma A/S, 2970 Hørsholm (DK)
(72) Inventor: HANSEN, John Bondo, 2100 Copenhagen Ø (DK); YOO, Hee-Won, Seoul 06955 (KR); RISWANTO, Riswanto, Seoul 06955 (KR)
(74) Representative: Høiberg P/S
(86) International application number: PCT/EP2019/086655
(87) International publication number: WO 2020/127954

(56) References cited:
- WO-A1-2012/163365
- WO-A1-2016/115223
- KAZUYA K ET AL: "TANDOSPIRONE CITRATE, A SELECTIVE 5-HT1A AGONIST, ALLEVIATES L-DOPA-INDUCED DYSKINESIA IN PATIENTS WITH PARKINSON'S DISEASEÜ", BRAIN AND NERVE / NO TO SHINKEI, IGAKU-SHOIN LTD., TOKYO, JP, vol. 54, no. 2, 1 February 2002 (2002-02-01), pages 133-137, XP001084533, ISSN: 0006-8969
- ESKOW ET AL: "The partial 5-HT"1"A agonist buspirone reduces the expression and development of l-DOPA-induced dyskinesia in rats and improves l-DOPA efficacy", PHARMACOLOGY BIOCHEMISTRY AND BEHAVIOR, ELSEVIER, US, vol. 87, no. 3, 27 July 2007 (2007-07-27), pages 306-314, XP022173598, ISSN: 0091-3057, DOI: 10.1016/J.PBB.2007.05.002
- DEKUNDY ET AL: "Modulation of l-DOPA-induced abnormal involuntary movements by clinically tested compounds: Further validation of the rat dyskinesia model", BEHAVIOURAL BRAIN RESEARCH, ELSEVIER, AMSTERDAM, NL, vol. 179, no. 1, 23 March 2007 (2007-03-23), pages 76-89, XP005935156, ISSN: 0166-4328

## Description

### Technical field

The present disclosure relates to optimised administration regimens of 5-HT1A agonists and levodopa in the management of movement disorders, such as Parkinson's disease and levodopa-induced dyskinesia (LID).

### Background

Movement disorders are a group of diseases that affect the ability to produce and control body movement, and are often associated with neurological disorders or conditions associated with neurological dysfunction. Movement disorders may manifest themselves in abnormal fluency or speed of movement, excessive or involuntary movement, or slowed or absent voluntary movement.

Movement disorders are frequently caused by impaired regulation of dopamine neurotransmission. Parkinson's disease (PD) is an example of a movement disorder associated with dysfunctional regulation of dopamine neurotransmission, which is caused by progressive degeneration of dopamine neurons. Tardive dyskinesia is another example of a movement disorder associated with dysfunctional regulation of dopamine neurotransmission.

In order to replace the lost dopamine, PD is currently treated with e.g. levodopa (L-DOPA, a precursor of dopamine). Unfortunately, the treatment of PD with L-DOPA often gives rise to a specific type of dyskinesia called L-DOPA Induced Dyskinesia (LID) which, in part, is caused by excessive dopamine levels in the synapses.

Dopamine release and re-uptake is regulated by a number of neurotransmitters, including serotonin (5-HT). Serotonin acts by binding to a number of different serotonergic receptors, of which agonists and antagonists of some serotonergic receptors have been investigated for treatment of movement disorders.

Modulators of serotonin (5-HT) neurotransmission individually have been shown to ameliorate or prevent LID. One example thereof is sarizotan, which is a 5-HT1A agonist and a dopamine receptor antagonist (Grégoire et al: *Parkinsonism Relat*

*Disord.* 2009; 15(6): 445-52). In pre-clinical and clinical studies sarizotan reduced LID, however, in phase 2b and3 studies no significant effects of sarizotan compared to placebo could be shown. Sarizotan has also been shown to have effects in a pre-clinical model of tardive dyskinesia (Rosegarten et al: Progress in Neuro-Psychopharmacology & Biological Psychiatry 30 (2006) 273 - 279). A selective antagonist of dopamine D4 receptors also reduced LID in a non-human primate model (P. Huot et al: JPET 342:576-585, 2012).

Buspirone and 5-HT1A agonists in general have been shown to reduce abnormal involuntary movements associated with L-DOPA treatment of Parkinson's disease (L-DOPA induced dyskinesia, LID) (for a review see e.g. P. Huot at al: Pharmacol Rev 65:171-222, 2013) and tardive dyskinesia (TD) associated with neuroleptic treatment of schizophrenia (e.g. Naidu et al: Eur J Pharmacol. 2001, 28; 428(1): 81-6; Creed et al: The Journal of Neuroscience, 2012, 32(28): 9574 -9581.

The effects of the 5-HT1A partial agonist buspirone on Parkinson's disease have been studied in a small open study (Ludwig et al: Clin Neuropharmacol. 1986; 9(4):373-8). It was found that doses (10-60 mg/day), which are normally used to treat patients suffering from anxiety, did not have any effects on Parkinson's disease or dyskinesia. At higher doses (100 mg/day) it was observed that buspirone reduced dyskinesia but with a significant worsening of disability ratings. This showed that high doses of buspirone could worsen the akinesia associated with Parkinson's disease. Other studies have shown that buspirone reduce L-DOPA induced dyskinesia in exploratory clinical studies (Bonifati et. al., 1994, Kleedorfer et al.,1991). Buspirone has furthermore been shown to have effects in clinical studies in tardive dyskinesia (Moss et. al., 1993).

5-HT1A agonists given in high doses can lead to the development of the serotonin syndrome or serotonin toxicity; a form of poisoning. Because of the severity of serotonin syndrome, it is therefore important to maintain a low exposure of the 5-HT1A agonist.

The serotonin syndrome is caused by increased activation of the 5-HT1A and 5-HT2A receptors. Serotonin syndrome, by definition, is a group of symptoms presenting as mental changes, autonomic nervous system malfunction, and neuromuscular complaints. Patients may present with confusion, agitation, diarrhoea, sweating, shivering, hypertension, fever, increased white blood cell count, incoordination, marked increase in reflexes, muscle jerks, tremor, extreme stiffness, seizures and even coma. The severity of changes ranges from mild to fatal.

In order to increase efficacy of 5-HT1A agonists in reducing LID in animal models a combination of a 5-HT1A and a 5-HT1B agonist has been tested (e.g. Muñoz et al: Brain. 2008; 131: 3380-94; Muñoz et al: Experimental Neurology 219 (2009) 298-307). The combined 5-HT1A and 5-HT1B agonist eltoprazine has also been suggested for treatment of LID (WO2009/156380) as well as an agonist of two or more the 5-HT1B, 5-HT1D and 5-HT1F receptors in combination with a 5-HT1A agonist when assayed in an animal model for LID, thus effectively increasing the therapeutic index (WO2012/048710).

Orally administrated buspirone undergoes extensive first pass metabolism, which limits bioavailability of the parent compound (4% in humans). This potentially will reduce the duration of action of the compound and necessitate the use of higher or multiple doses. Buspirone is metabolized through cytochrome P450 enzymes. This in turn may increase the risk of drug-drug interactions, which is particularly relevant for patients with movement disorders who often receive more than one medicament.

### Summary

The present invention is defined by the claims.

The present inventors aim to achieve a better relationship between on one side the efficacy of 5-HT1A agonists and on the other side the observed side effects associated with efficacious dosages of 5-HT1A agonists. This may be achieved with multiple dosages or an administration form resembling multiple dosages, such as a continuous administration form, of 5-HT1A agonists and/or may be achieved with a more simultaneous administration of 5-HT1A agonists with L-DOPA preparations to obtain a higher concurrent exposure of the two compounds.

The present disclosure thus relates to a composition for use in the treatment, prevention or alleviation of a movement disorder. The composition of the present disclosure comprises a 5-HT1A receptor agonist, or a pharmaceutically acceptable derivative thereof, and may further comprise, separately or together, dopamine, a dopamine agonist, a dopamine precursor. The composition of the present disclosure does not comprise an agonist of one or more of the serotonin receptors selected from the group of 5-HT1B, 5-HT1D and/or 5-HT1F receptor, or a pharmaceutically acceptable derivative thereof.

### Definitions

The term "agonist" in the present context refers to a substance capable of binding to and activating a (one or more) receptor(s). A 5-HT1A receptor agonist (5-HT1A agonist) is thus capable of binding to and activating the 5-HT1A receptor. An agonist of two or more the 5-HT1B, 5-HT1D and 5-HT1F receptors (5-HT1B/D/F agonist) is capable of binding to and activating two or three of the 5-HT1B, 5-HT1D and 5-HT1F receptors. The terms `5-HT1 agonist', `5-HT1 receptor agonist', and 'agonist of the 5-HT1 receptor' are used interchangeably herein.

"Partial agonists" in the present context are compounds able to bind and activate a given receptor, but having only partial efficacy at the receptor relative to a "full agonist". Partial agonists can act as antagonists when competing with a full agonist for receptor occupancy and producing a net decrease in the receptor activation compared to the effects or activation observed with the full agonist alone.

The term "antagonist" in the present context refers to a substance capable of inhibiting the effect of a receptor agonist.

The terms "dopamine," "DA" and "4-(2-aminoethyl)benzene-1,2-diol," refer to a catecholamine neurotransmitter and hormone. Dopamine is a precursor of adrenaline (epinephrine) and noradrenaline (norepinephrine) and activates the five types of dopamine receptors - D1, D2, D3, D4, and D5 - and their variants.

"L-DOPA" or "3,4-dihydroxyphenylalanine" is a precursor to the neurotransmitters dopamine, norepinephrine (noradrenaline), and epinephrine (adrenaline). L-DOPA is able to cross the blood-brain barrier, and is converted to dopamine by the enzyme aromatic L-amino acid decarboxylase (AADC), also known as DOPA decarboxylase (DDC). L-DOPA is used for treatment of Parkinson's disease.

The terms "Parkinson's disease," "Parkinson's" and "PD" refer to a neurological syndrome characterized by a dopamine deficiency, resulting from degenerative, vascular, or inflammatory changes in the basal ganglia of the substantia nigra. This term also refers to a syndrome which resembles Parkinson's disease, but which may or may not be caused by Parkinson's disease, such as Parkinsonian-like side effects caused by certain antipsychotic drugs. Parkinson's disease is also referred to as paralysis agitans and shaking palsy.

The terms "serotonin," "5-hydroxytryptamine" and "5-HT" refers to a phenolic amine neurotransmitter produced from tryptophan by hydroxylation and decarboxylation in serotonergic neurons of the central nervous system and enterochromaffin cells of the gastrointestinal tract. Serotonin is a precursor of melatonin.

The term "pharmaceutically acceptable derivative" in present context includes pharmaceutically acceptable salts, which indicate a salt which is not harmful to the patient. Such salts include pharmaceutically acceptable basic or acid addition salts as well as pharmaceutically acceptable metal salts, ammonium salts and alkylated ammonium salts. A pharmaceutically acceptable derivative further includes esters and prodrugs, or other precursors of a compound which may be biologically metabolized into the active compound, or crystal forms of a compound.

The term "therapeutically effective amount" of a compound as used herein refers to an amount sufficient to cure, alleviate, prevent, reduce the risk of, or partially arrest the clinical manifestations of a given disease or disorder and its complications.

The terms "treatment" and "treating" as used herein refer to the management and care of a patient for the purpose of combating a condition, disease or disorder. The term is intended to include the full spectrum of treatments for a given condition from which the patient is suffering, such as administration of the active compound for the purpose of: alleviating or relieving symptoms or complications; delaying the progression of the condition, disease or disorder; curing or eliminating the condition, disease or disorder; and/or preventing the condition, disease or disorder, wherein "preventing" or "prevention" is to be understood to refer to the management and care of a patient for the purpose of hindering the development of the condition, disease or disorder, and includes the administration of the active compounds to prevent or reduce the risk of the onset of symptoms or complications. The patient to be treated is preferably a mammal, in particular a human being.

A "triptan" in the present context is a compound part of a family of tryptamine-based drugs used as abortive medication in the treatment of migraines and cluster headaches. The triptans are agonists of several of the serotonin receptors (such as two or more), with varying potency for the different 5-HT1 receptor subtypes, primarily 5-HT1B, 5-HT1D, 5-HT1E and/or 5-HT1F receptors.

### Detailed description

A composition according to the present disclosure is in one embodiment a pharmaceutical composition, a pharmaceutically acceptable composition and/or a pharmaceutically safe composition. A composition according to the present disclosure comprises at least a 5-HT1A receptor agonist as active ingredient.

It is an aspect of the present disclosure to provide a pharmaceutical composition comprising a 5-HT1A receptor agonist, or a pharmaceutically acceptable derivative thereof,
wherein said composition optionally further comprises, separately or together, dopamine, a dopamine agonist, a dopamine precursor. with the proviso that said composition does not comprise an agonist of one or more of the serotonin receptors selected from the group of 5-HT1B, 5-HT1D and/or 5-HT1F receptor, or a pharmaceutically acceptable derivative thereof, for use in the treatment, prevention or alleviation of a movement disorder, wherein the composition is to be administered at least two times daily.

Also disclosed herein is a combination therapy for the treatment, prevention or alleviation of a movement disorder comprising, separately or together, i) 5-HT1A receptor agonist, or a pharmaceutically acceptable derivative thereof, and ii) dopamine, a dopamine agonist, a dopamine precursor

It is thus an aspect to provide such composition as disclosed herein wherein said composition invariably further comprises, separately or together, dopamine, a dopamine agonist, a dopamine precursor or a dopamine prodrug.

In certain embodiments the compositions as disclosed herein consist of a 5-HT1A receptor agonist, or a pharmaceutically acceptable derivative thereof, and dopamine, a dopamine agonist, a dopamine precursor as the sole active pharmaceutical ingredients.

It is thus an aspect to provide a pharmaceutical composition comprising a 5-HT1A receptor agonist, or a pharmaceutically acceptable derivative thereof, wherein said composition further comprises, separately or together, dopamine, a dopamine agonist, a dopamine precursor with the proviso that said composition does not comprise an agonist of one or more of the serotonin receptors selected from the group of 5-HT1B, 5-HT1D and/or 5-HT1F receptor, or a pharmaceutically acceptable derivative thereof, for use in the treatment, prevention or alleviation of a movement disorder.

In one embodiment the composition for use as disclosed herein comprises or consists of, together, a i) 5-HT1A receptor agonist, or a pharmaceutically acceptable derivative thereof, and ii) dopamine, a dopamine agonist, a dopamine precursor

A composition comprising together more than one compound is meant to encompass embodiments wherein the two or more compounds are included within the same single pharmaceutical composition, wherein said pharmaceutical composition may have any form as known to the skilled person.

In one embodiment the composition for use as disclosed herein comprises or consists of, separately, a i) 5-HT1A receptor agonist, or a pharmaceutically acceptable derivative thereof, and ii) dopamine, a dopamine agonist, a dopamine precursor

A composition comprising separately more than one compound is meant to encompass embodiments wherein the two or more compounds are included within separate or distinct pharmaceutical compositions, or different compartments of a composite pharmaceutical composition, wherein said pharmaceutical composition may have any form as known to the skilled person.

### Combination administration

It is an aspect of the present disclosure to provide a pharmaceutical composition comprising, separately or together, i) 5-HT1A receptor agonist, or a pharmaceutically acceptable derivative thereof, and ii) dopamine, a dopamine agonist, a dopamine precursor.

It is understood that for a composition comprising multiple compounds, each of said compounds may be administered simultaneously, separately or sequentially.

In one embodiment there is provided a pharmaceutical composition comprising, separately or together, i) 5-HT1A receptor agonist, or a pharmaceutically acceptable derivative thereof, and ii) dopamine, a dopamine agonist, a dopamine precursor, with the proviso that said composition does not comprise an agonist of one or more of the serotonin receptors selected from the group of 5-HT1B, 5-HT1D and/or 5-HT1F receptor, or a pharmaceutically acceptable derivative thereof,
for use in the treatment, prevention or alleviation of a movement disorder,

wherein said 5-HT1A receptor agonist, or a pharmaceutically acceptable derivative thereof, is to be administered simultaneously, separately or sequentially with said dopamine, dopamine agonist, dopamine precursor.

In one embodiment said composition is to be administered more than once daily, such as at least 2 times daily, at least 3 times daily, at least 4 times daily, at least 5 times daily, at least 6 times daily, at least 7 times daily or at least 8 times daily.

In embodiments wherein said composition is to be administered more than once daily, said 5-HT1A receptor agonist, or a pharmaceutically acceptable derivative thereof, is to be administered simultaneously, separately or sequentially with said dopamine, dopamine agonist, dopamine precursor for each administration.

In one embodiment said 5-HT1A receptor agonist, or a pharmaceutically acceptable derivative thereof, and said dopamine, a dopamine agonist, a dopamine precursor or a dopamine prodrug, is to be administered simultaneously for each administration.

In one embodiment said 5-HT1A receptor agonist, or a pharmaceutically acceptable derivative thereof, and said dopamine, a dopamine agonist, a dopamine precursor, is to be administered separately or sequentially for each administration.

In one embodiment said 5-HT1A receptor agonist, or a pharmaceutically acceptable derivative thereof, and said dopamine, a dopamine agonist, a dopamine precursor, is to be administered essentially at the same time.

In one embodiment said 5-HT1A receptor agonist, or a pharmaceutically acceptable derivative thereof, and said dopamine, a dopamine agonist, a dopamine precursor, is to be administered essentially not separated in time.

'Essentially at the same time' will encompass any simultaneous, separate or sequential administration of i) a 5-HT1A receptor agonist, or a pharmaceutically acceptable derivative thereof, and ii) dopamine, a dopamine agonist, a dopamine precursor, which occurs without delay, or without intended delay, yet allowing sufficient time for performing said simultaneous, separate or sequential administration of the two compounds.

### Multiple dosages

In certain embodiments the compositions as disclosed herein are to be administered in multiple dosages, or any other administration form that resembles a multiple dosing regimen.

It is thus an aspect to provide a pharmaceutical composition comprising a 5-HT1A receptor agonist, or a pharmaceutically acceptable derivative thereof, wherein said composition optionally further comprises, separately or together, dopamine, a dopamine agonist, a dopamine precursor. with the proviso that said composition does not comprise an agonist of one or more of the serotonin receptors selected from the group of 5-HT1B, 5-HT1D and/or 5-HT1F receptor, or a pharmaceutically acceptable derivative thereof,
for use in the treatment, prevention or alleviation of a movement disorder,
wherein said composition is to be administered at least 2 times daily.

In one embodiment said composition is to be administered at least 3 times daily.

In one embodiment said composition is to be administered at least 4 times daily.

In one embodiment said composition is to be administered at least 5 times daily.

In one embodiment said composition is to be administered at least 6 times daily, at least 7 times daily or at least 8 times daily.

In one embodiment said composition is to be administered 2 times daily, such as 3 times daily, such as 4 times daily, such as 5 times daily, such as 6 times daily, such as 7 times daily, such as 8 times daily, or more.

In one embodiment said composition is to be administered 2-3 times daily, such as 3-4 times daily, such as 4-5 times daily, such as 5-6 times daily, such as 6-7 times daily, such as 7-8 times daily, or more.

In certain embodiments the compositions as disclosed herein are to be administered in an administration form that resembles a multiple dosing regimen.

A multiple dosing regimen may allow for administration of a lower dosage per administration, which in turn may result in retaining a lower and constant concentration in the blood.

It is thus an aspect to provide a pharmaceutical composition comprising a 5-HT1A receptor agonist. wherein said composition optionally further comprises, separately or together, dopamine, a dopamine agonist, a dopamine precursor, with the proviso that said composition does not comprise an agonist of one or more of the serotonin receptors selected from the group of 5-HT1B, 5-HT1D and/or 5-HT1F receptor, or a pharmaceutically acceptable derivative thereof,
for use in the treatment, prevention or alleviation of a movement disorder, wherein said composition is to be administered:
i) as a continuous administration,
ii) as a depot injection, or
iii) as an extended release formulation.

### Composition comprising compounds

The present disclosure provides use of a pharmaceutical composition, or simply a composition, that comprises
i) a 5-HT1A receptor agonist, and
ii) optionally dopamine, a dopamine agonist, a dopamine precursor,
with the proviso that said composition does not comprise an agonist of one or more of the serotonin receptors selected from the group of 5-HT1B, 5-HT1D and/or 5-HT1F receptor, or a pharmaceutically acceptable derivative thereof, as defined herein.

In one embodiment said composition does not comprise an agonist of one or more of the serotonin receptors selected from the group of 5-HT1B, 5-HT1D and/or 5-HT1F receptor.

In one embodiment said composition does not comprise an agonist of two or more of the 5-HT1B, 5-HT1D and 5-HT1F receptors.

In one embodiment said composition does not comprise a triptan.

In one embodiment said composition does not comprise a triptan selected from the group consisting of zolmitriptan, rizatriptan, sumatriptan, naratriptan, almotriptan, frovatriptan, avitriptan, imotriptan and eletriptan.

### Compound i) a 5-HT1A receptor agonist

In one embodiment, the compound i) 5-HT1A agonist is a full agonist or a partial agonist.

In one embodiment the 5-HT1A agonist as used herein is selected from the group consisting of buspirone (8-[4-(4-pyrimidin-2-ylpiperazin-1-yl)butyl]-8-azaspiro[4.5]decane-7,9-dione), tandospirone ((1R,2R,6S,7S)-4-{4-[4-(pyrimidin-2-yl)piperazin-1-yl]butyl}-4-azatricyclo[5.2.1.02,6]decane-3,5-dione), gepirone (4,4-dimethyl-1-[4-(4-pyrimidin-2-ylpiperazin-1-yl)butyl]piperidine-2,6-dione), alnespirone ((+)-4-dihydro-2H-chromen-3-yl]-propylamino]butyl]-8-azaspiro[4.5]decane-7,9-dione), binospirone (8-[2-(2,3-dihydro-1,4-benzodioxin-2-ylmethylamino)ethyl]-8-azaspiro[4.5]decane-7,9-dione), ipsapirone (9,9-dioxo-8-[4-(4-pyrimidin-2-ylpiperazin-1-yl)butyl]-9λ6-thia-8-azabicyclo[4.3.0]nona-1,3,5-trien-7-one), perospirone (3a*R*, 7a*S*)-2-{4-[4-(1, 2-benzisothiazol-3-yl)piperazin-1-yl]butyl} hexahydro-1*H*-isoindole-1,3(2*H*)-dione, befiradol (F-13,640) (3-chloro-4-fluorophenyl-[4-fluoro-4-([(5-methylpyridin-2-yl)methylamino]methyl)piperidin-1-yl]methanone, repinotan ((R)-(-)-2-[4-[(chroman-2-ylmethyl)-amino]-butyl]-1,1-dioxo-benzo[d] isothiazolone), piclozotan (3-chloro-4-[4-[4-(2-pyridinyl)-1,2,3,6-tetrahydropyridin-1-yl]butyl]-1,4-benzoxazepin-5(4H)-one), osemozotan (5-(3-[((2S)-1,4-benzodioxan-2-ylmethyl)amino]propoxy)-1,3-benzodioxole), flesinoxan (4-fluoro-N-[2-[4-[(3S)-3-(hydroxymethyl)-2,3-dihydro-1,4-benzodioxin-8-yl]piperazin-1-yl]ethyl]benzamide), flibanserin (1-(2-{4-[3-(trifluoromethyl)phenyl]piperazin-1-yl}ethyl)-1,3-dihydro-2H-benzimidazol-2-one), and sarizotan (EMD-128,130) (1-[(2R)-3,4-dihydro-2H-chromen-2-yl]-N-([5-(4-fluorophenyl)pyridin-3-yl]methyl)methanamine), or a pharmaceutically acceptable derivative thereof.

Thus, in a preferred embodiment, the 5-HT1A agonist is selected from the group consisting of buspirone, tandospirone, gepirone, alnespirone, binospirone, ipsapirone, perospirone, befiradol, repinotan, piclozotan, osemozotan, flesinoxan, flibanserin, sarizotan, eltoprazine, F13714 and F15599 and pharmaceutically acceptable derivatives thereof.

In a particular embodiment said 5-HT1A agonist is buspirone, tandospirone or gepirone. In another particular embodiment said 5-HT1A agonist is buspirone or tandospirone. In yet another particular embodiment said 5-HT1A agonist is buspirone.

Buspirone (8-[4-(4-pyrimidin-2-ylpiperazin-1-yl)butyl]-8-azaspiro[4.5]decane-7,9-dione) is a drug of the azapirone chemical class approved for treatment of anxiety disorders. Buspirone is a serotonin 5-HT1A receptor partial agonist, which is thought to mediate its anxiolytic and antidepressant effects. Additionally, it is a presynaptic dopamine antagonist at the D2, D3 and D4 receptors, and a partial α₁ receptor agonist.

### Compound ii) dopamine, a dopamine agonist, a dopamine precursor or a dopamine prodrug

The present disclosure provides use of a pharmaceutical composition, or simply a composition, that comprises
i) a 5-HT1A receptor agonist, and
ii) dopamine, a dopamine agonist, a dopamine precursor.

In one embodiment the compound ii) is an agent that increases the dopamine concentration in the synaptic cleft.

In one embodiment the compound ii) is selected from the group consisting of dopamine, a dopamine agonist, a dopamine precursor.

In one embodiment the compound ii) is selected form the group consisting of dopamine, a dopamine precursor. A dopamine precursor is a substance that can be converted into dopamine in the body.

In one embodiment the compound ii) is dopamine.

In one embodiment the compound ii) is a dopamine precursor or dopaminergic precursor. Known dopamine precursors include L-phenylalanine, L-tyrosine and L-DOPA.

In a preferred embodiment the compound ii) is L-DOPA (levodopa; L-3,4-dihydroxyphenylalanine). Brand names include Dopar and Larodopa.

Alternatively, the compound ii) may be an L-DOPA derivative, such as a deuterated analogue of L-DOPA or an ester of L-DOPA.

L-DOPA crosses the protective blood-brain barrier, whereas dopamine itself cannot. Thus, L-DOPA is used to increase dopamine concentrations in the treatment of Parkinson's disease and dopamine-responsive dystonia.

It is understood that any formulation or composition comprising L-DOPA, such as any commercially available formulation or composition comprising L-DOPA, is meant to be encompassed by the present disclosure. These include solid preparations, both immediate and extended release, as well as liquid preparations.

In humans, conversion of L-DOPA to dopamine does not only occur within the central nervous system. Cells in the peripheral nervous system perform the same task. Thus, administering L-DOPA alone will lead to increased dopamine signalling in the periphery as well. Excessive peripheral dopamine signalling is undesirable as it causes many of the adverse side effects seen with sole L-DOPA administration.

To avoid peripheral conversion of L-DOPA to dopamine it is standard clinical practice to co-administer with L-DOPA a peripheral DOPA decarboxylase inhibitor (DDCI).

In one embodiment the compound ii) is a formulation or composition comprising L-DOPA, including any commercially available formulation or composition comprising L-DOPA, optionally in combination with one or more further active ingredients.

In one embodiment the compound ii) L-DOPA or a formulation or composition comprising L-DOPA is selected from the group consisting of:
i) L-DOPA in combination with a DOPA decarboxylase inhibitor, including
   a. L-DOPA and carbidopa (levocarb or co-careldopa);
      i. Such as Sinemet (Atamet, Carbilev), pharmacope, parcopa and madopar,
      ii. Such as L-DOPA with Lodosyn (carbidopa)
   b. L-DOPA and benserazide,
      i. Such as Madopar or Prolopa
ii) L-DOPA in combination with a COMT inhibitor (catecholamine-O-methyltransferase),
   a. Including L-DOPA and tolcapone; and L-DOPA and entacapone, L-DOPA and opicapone, and L-DOPA and nitecapone,
iii) L-DOPA in combination with a decarboxylase inhibitor and a COMT inhibitor
   a. Such as Stalevo (Carbidopa/levodopa/entacapone), including Stalevo 50, 75, 100, 125, 150 and 200.

In another embodiment the compound ii) is a dopamine agonist.

In one embodiment the compound ii) is a dopamine receptor agonist.

A dopamine receptor agonist is a compound that activates dopamine receptors. Dopamine receptor agonists activate signalling pathways through trimeric G-proteins and β-arrestins, ultimately leading to changes in gene transcription. Agonists are available for several dopamine receptor subtypes (D1, D2, D3) that differentially address these signalling pathways, called biased agonists.

In one embodiment the compound ii) is a biased dopamine agonist, a partial dopamine agonist, a selective dopamine agonist, or a full dopamine agonist.

In one embodiment the compound ii) is a dopamine receptor agonist selected from the group consisting of bromocriptine (Parlodel), pergolide (Permax), pramipexole (Mirapex, Sifrol), ropinirole (Requip), piribedil (Pronora, Trivastal), cabergoline (Dostinex), apomorphine (Apokyn), propylnorapomorphine, lisuride, Ciladopa, dihydrexidine, dinapsoline, doxanthrine, epicriptine, quinagolide (Norprolac), rotigotine (Neupro), roxindole, sumanirole, fenoldopam, and derivatives thereof.

In one embodiment the compound ii) is an indirect dopamine agonist. In one embodiment the indirect dopamine agonist is selected from the group consisting of a dopamine reuptake inhibitor / transporter blocker and dopamine releasing agent.

In one embodiment the indirect dopamine agonist is selected from the group consisting of amphetamine and/or dextroamphetamine, Bupropion (Wellbutrin), Amineptine, nomifensine, Lisdexamfetamine, Methylphenidate (Ritalin) or dexmethylphenidate, methylenedioxypyrovalerone (MDPV; "Sonic"), ketamine, and phencyclidine (PCP), Cathinone, Cocaine, amphetamine, Methamphetamine, methylenedioxymethamphetamine (MDMA), Phenethylamine, p-Tyramine, lisdexamfetamine (Vyvanse), phenmetrazine, pemoline, 4-methylaminorex (4-MAR), and benzylpiperazine, and Vesicular monoamine transporter 2 (VMAT2) inhibitors such as reserpine, tetrabenazine, and deserpidine.

In one embodiment the present disclosure provides the use of a pharmaceutical composition comprising buspirone and L-DOPA (levodopa).

The composition of the present disclosure does not comprise an agonist of one or more of the serotonin receptors selected from the group of 5-HT1B, 5-HT1D and/or 5-HT1F receptor, or a pharmaceutically acceptable derivative thereof.

An agonist of one or more of the 5-HT1B, 5-HT1D and 5-HT1F receptors may be an agonist of one, two or three serotonin receptors selected from the group consisting of 5-HT1B, 5-HT1D, and 5-HT1F receptors. Thus it may be an agonist of one of the 5-HT1B, 5-HT1D and 5-HT1F receptors, whereas in other embodiments it is a combined agonist of the 5-HT1B receptor and 5-HT1D receptor, or a combined agonist of the 5-HT1B receptor and 5-HT1F receptor, or a combined agonist of the 5-HT1D receptor and 5-HT1F receptor, or a combined agonist of the 5-HT1B receptor, the 5-HT1D receptor and the 5-HT1F receptor. It may also have some agonistic activity on the 5-HT1A receptor (full or partial).

In one embodiment the agonist of one or more of the 5-HT1B, 5-HT1D and 5-HT1F receptors is an agonist of two or more of the 5-HT1B, 5-HT1D and 5-HT1F receptors. In one embodiment the agonist of two or more of the 5-HT1B, 5-HT1D and 5-HT1F receptors is a triptan. A "triptan" in the present context is a compound part of a family of tryptamine-based drugs used as abortive medication in the treatment of migraines and cluster headaches. The triptans are agonists of several of the serotonin receptors, with varying potency for the different 5-HT1 receptor subtypes, primarily 5-HT1B, 5-HT1D, 5-HT1E and/or 5-HT1F receptors.

In one embodiment the agonist of one or more, such as two or more, of the 5-HT1B, 5-HT1D and 5-HT1F receptors is selected from the group consisting of zolmitriptan ((S)-4-({3-[2-(dimethylamino)ethyl]-1H-indol-5-yl}methyl)-1,3-oxazolidin-2-one), rizatripan (N,N-dimethyl-2-[5-(1H-1,2,4-triazol-1-ylmethyl)-1H-indol-3-yl]ethanamine), sumatriptan (1-[3-(2-dimethylaminoethyl)-1H-indol-5-yl]- N-methyl-methanesulfonamide), naratripan (N-methyl-2-[3-(1-methylpiperidin-4-yl)-1H-indol-5-yl]ethanesulfonamide), almotriptan (N,N-dimethyl-2- [5-(pyrrolidin-1-ylsulfonylmethyl)- 1H-indol-3-yl]-ethanamine), frovatriptan ((+)-(R)-3-methylamino-6-carboxamido-1,2,3,4-tetrahydrocarbazole) and eletriptan ((R)-3-[(-1-methylpyrrolidin-2-yl)methyl]-5-(2-phenylsulfonylethyl)- 1H-indole), or a pharmaceutically acceptable derivative thereof. The triptan may be selected from the group consisting of zolmitriptan, rizatriptan, sumatriptan, naratriptan, almotriptan, frovatriptan, avitriptan, imotriptan, alniditan and eletriptan.

The triptan may also be selected from the group consisting of zolmitriptan, rizatriptan, sumatriptan, naratriptan, almotriptan, frovatriptan, avitriptan, imotriptan and eletriptan.

The triptan may also be zolmitriptan, rizatripan, frovatriptan, eletriptan or naratriptan. Zolmitriptan, rizatriptan, naratriptan and eletriptan are full agonists of 5-HT1D, B and A, and partial agonists of 5-HT1B.

### Movement disorders

The present disclosure relates to a composition comprising a 5-HT1A receptor agonist, or a pharmaceutically acceptable derivative thereof, and optionally dopamine, a dopamine agonist, a dopamine precursor or a dopamine prodrug, as defined herein, for use in the treatment of movement disorders. The term treatment includes treatment, prevention / prophylaxis (reduction of risk) and amelioration.

In one embodiment the movement disorder is a disorder associated with altered or impaired synaptic dopamine levels.

In one embodiment, the movement disorder according to the present disclosure is selected from the group consisting of Parkinson's disease, movement disorders associated with Parkinson's disease, bradykinesia, akinesia, dyskinesia, L-DOPA induced dyskinesia, tardive dyskinesia, ataxia, akathisia, dystonia, essential tremor, Huntington's disease, myoclonus, Rett syndrome, Tourette syndrome, Wilson's disease, chorea, Machado-Joseph disease, restless leg syndrome, spasmodic torticollis, geniospasm, or movement disorders associated therewith.

Parkinson's disease is associated with muscle rigidity, tremor, postural abnormalities, gait abnormalities, a slowing of physical movement (bradykinesia), and in extreme cases a loss of physical movement (akinesia). PD is caused by degeneration and death of dopaminergic neurons in substantia nigra pars compacta and leads to dysfunctional regulation of dopamine neurotransmission.

In one particular embodiment of the present disclosure the movement disorder is Parkinson's disease. In one particular embodiment of the present disclosure the movement disorder is Parkinson's disease or the associated movement disorders akinesia, dyskinesia and bradykinesia, or movement disorders associated with Parkinson's disease or treatment of Parkinson's disease such as L-DOPA induced dyskinesia (LID).

In one particular embodiment of the present disclosure the movement disorder is L-DOPA induced dyskinesia (LID).

In another embodiment of the present disclosure, the movement disorder is caused by idiopathic disease, genetic dysfunctions, infections or other conditions which lead to dysfunction of the basal ganglia and/or lead to altered synaptic dopamine levels.

Movement disorders according to the present disclosure are in one embodiment associated with the use of medications or drugs / drug therapy.

In one embodiment of the present disclosure, the movement disorder is associated with or caused by treatment of Parkinson's disease, such as antiparkinsonian therapy, including but not limited to dopamine, dopamine agonists, dopamine precursors, dopamine prodrugs, dopamine mimetics and dopaminergic drugs, such as for example L-DOPA.

In one embodiment of the present disclosure, the movement disorder is associated with or caused by neuroleptic drugs, antipsychotics, antidepressants and anti-emetic drugs.

In one embodiment the movement disorder is caused by or associated with the use or medication of antipsychotics such as haloperidol, droperidol, pimozide, trifluoperazine, amisulpride, risperidone, aripiprazole, asenapine, and zuclopenthixol, antidepressants such as fluoxetine, paroxetine, venlafaxine, and trazodone, anti-emetic drugs such as dopamine blockers for example metoclopramide (reglan) and prochlorperazine (compazine).

In yet another embodiment of the present disclosure, the movement disorder is caused by or associated with withdrawal of opioids, barbiturates, cocaine, benzodiazepines, alcohol, or amphetamines.

It is an aspect of the present disclosure to provide a composition as defined herein for use in a method for the treatment of a movement disorder.

It is an aspect of the present disclosure to provide a composition as defined herein for manufacture of a medicament for the treatment of a movement disorder.

In one embodiment, the composition as defined herein for use in a method for the treatment of a movement disorder is administered to an individual in need thereof.

An individual in need as referred to herein, is an individual that may benefit from the administration of a compound or pharmaceutical composition according to the present disclosure. Such an individual may suffer from a movement disorder or be in risk of suffering from a movement disorder. The individual may be any human being, male or female, infant, middle-aged or old. The movement disorder to be treated or prevented in the individual may relate to the age of the individual, the general health of the individual, the medications used for treating the individual and whether or not the individual has a prior history of suffering from diseases or disorders that may have or have induced movement disorders in the individual.

The present disclosure relates to the treatment of an individual having a risk (e.g. an increased risk) of suffering from a movement disorder. In one embodiment said individual having a risk of suffering from a movement disorder is a person which is, or is to be, treated with a dopamine prodrug such as L-DOPA (e.g. levodopa).

It is an embodiment of the present disclosure to provide a pharmaceutical composition comprising a 5-HT1A receptor agonist, or a pharmaceutically acceptable derivative thereof, and comprising, separately or together, dopamine, a dopamine agonist, a dopamine precursor or a dopamine prodrug,
with the proviso that said composition does not comprise an agonist of one or more of the serotonin receptors selected from the group of 5-HT1B, 5-HT1D and/or 5-HT1F receptor, or a pharmaceutically acceptable derivative thereof,
for use in the treatment, prevention or alleviation of L-DOPA induced dyskinesia (LID).

In one embodiment said composition comprises a 5-HT1A agonist selected from the group consisting of buspirone, 6-hydroxybuspirone, tandospirone, gepirone, alnespirone, binospirone, ipsapirone, perospirone, befiradol, repinotan piclozotan, osemozotan, flesinoxan, flibanserin, sarizotan, eltoprazine, F13714 and F15599, and L-DOPA, for use in the treatment, prevention or alleviation of L-DOPA induced dyskinesia (LID).

In one embodiment said composition comprises buspirone and L-DOPA for use in the treatment, prevention or alleviation of L-DOPA induced dyskinesia (LID).

In one embodiment said composition comprises 6-hydroxybuspirone and L-DOPA for use in the treatment, prevention or alleviation of L-DOPA induced dyskinesia (LID).

In one embodiment said composition comprises a 5-HT1A agonist selected from the group consisting of buspirone, tandospirone, gepirone, alnespirone, binospirone, ipsapirone, perospirone, befiradol, repinotan piclozotan, osemozotan, flesinoxan, flibanserin, sarizotan, eltoprazine, F13714 and F15599, and L-DOPA, for use in the treatment, prevention or alleviation of L-DOPA induced dyskinesia (LID), wherein said composition is to be administered at least 2 times daily, at least 3 times daily, at least 4 times daily, at least 5 times daily, at least 6 times daily, at least 7 times daily or at least 8 times daily.

It is a further embodiment of the present disclosure to provide a pharmaceutical composition comprising a 5-HT1A receptor agonist, or a pharmaceutically acceptable derivative thereof,
wherein said composition optionally further comprises, separately or together, dopamine, a dopamine agonist, a dopamine precursor,
with the proviso that said composition does not comprise an agonist of one or more of the serotonin receptors selected from the group of 5-HT1B, 5-HT1D and/or 5-HT1F receptor, or a pharmaceutically acceptable derivative thereof,
for use in the treatment, prevention or alleviation of tardive dyskinesia (TD), wherein said composition is to be administered at least 5 times daily.

### Administration and dosage

It will be appreciated that the preferred route of administration will depend on the general condition and age of the subject to be treated, the nature of the condition to be treated, the location of the tissue to be treated in the body and the active ingredient(s). In one embodiment the route of administration allows for the compound or composition to cross the blood-brain barrier.

Systemic treatment according to the present disclosure is capable of introducing the compound or composition into the blood stream to ultimately target the sites of desired action.

Systemic treatment includes administration via the enteral route and the parenteral route including oral, rectal, nasal, vaginal, rectal, pulmonary, bronchial, buccal, sublingual, transdermal, topical, intracisternal, intraperitoneal, subcutaneous, intramuscular, intrathecal, intravenous and intradermal administration. Appropriate dosage forms for such administration may be prepared by conventional techniques.

The compound or composition according to the disclosure may be used as a local treatment, i.e. be introduced directly to the site(s) of action. Accordingly, the composition may be applied to the skin or mucosa directly, or the composition may be injected into the site of action, for example into the diseased tissue or to an end artery leading directly to the diseased tissue (intracavernous, intravitreal, intra-articular, intracerebral, intrathecal, epidural).

The pharmaceutical composition as disclosed herein may be administered one or several times per day, such as from 1 to 2 times per day, such as from 2 to 3 times per day, such as from 3 to 4 times per day, such as from 4 to 5 times per day, such as from 5 to 6 times per day, such as from 6 to 7 times per day, such as from 7 to 8 times per day. In one embodiment, the composition is administered once a day, such as twice per day, for example 3 times per day, such as 4 times per day, for example 5 times per day, such as 6 times per day.

In a particular embodiment, the composition is administered at least twice per day, for example at least 3 times per day, such as at least 4 times per day, for example at least 5 times per day, such as at least 6 times per day, or more.

Administration may occur for a limited time, such as from 1 or 2 days to 7 days, for example 7 days to 14 days, such as from 14 days to a month, for example from a month to several months (2, 3, 4, 5, 6, 7, 8, 9, 10, 11, 12 months); or administration may be chronic, the treatment may be chronic from the onset of diagnosis, such as throughout the lifetime of the individual or as long as the individual will benefit therefrom i.e. when a movement disorder is present or while having an increased risk of developing a movement disorder, such as during treatment with L-DOPA or other medications such as antipsychotics, antidepressants, anti-emetic drugs or during withdrawal of certain medications causing a movement disorder.

In one embodiment, the pharmaceutical formulation is to be administered as long as a movement disorder is present or as long as an increased risk of developing a movement disorder is present.

### Dosages

The concentration of each of the active ingredients in the present pharmaceutical composition namely a 5-HT1A agonist and optionally dopamine, a dopamine agonist, a dopamine precursor or a dopamine prodrug are optimized to achieve an appropriate dosage of each drug.

In one embodiment said 5-HT1A receptor agonist, or pharmaceutically acceptable derivative thereof, is administered at 0.1 to 100 mg per dosage, such as 0.1-1 mg, such as 1-5 mg, such as 5-10 mg, such as 10-15 mg, such as 15-20 mg, such as 20-25 mg, such as 25-30 mg, such as 30-35 mg, such as 35-40 mg, such as 40-45 mg, such as 45-50 mg, such as 50-75 mg, such as 75 to 100 mg per dosage.

In one embodiment said 5-HT1A receptor agonist, or pharmaceutically acceptable derivative thereof, is administered at a daily dosage of 0.01 to 10 mg/kg bodyweight, such as 0.01 to 5 mg/kg bodyweight, such as 0.1 to 1 mg/kg bodyweight.

In one embodiment said 5-HT1A receptor agonist, or pharmaceutically acceptable derivative thereof, is administered at a daily dosage of of from 0.001 to 100 mg/kg bodyweight, such as 0.001 to 0.005 mg/kg, for example 0.005 to 0.01 mg/kg, such as 0.01 to 0.05 mg/kg, for example 0.05 to 0.1 mg/kg, such as 0.1 to 0.5 mg/kg, for example 0.5 to 1.0 mg/kg, such as 1 to 2 mg/kg, for example 2 to 5 mg/kg, such as 5 to 10 mg/kg, for example 10 to 15 mg/kg, such as 15 to 20 mg/kg, for example 20 to 30 mg/kg, such as 30 to 40 mg/kg, for example 50 to 75 mg/kg, such as 75 to 100 mg/kg bodyweight.

L-DOPA is often administered at an initial dosage and later administered at a maintenance dosage.

In one embodiment said L-DOPA, or pharmaceutically acceptable derivative thereof, is administered at 100 to 10000 mg/day, such as 100-250 mg, such as 250-500 mg, such as 500-750 mg, such as 750-1000 mg, such as 1000-2000 mg, such as 2000-2500 mg, such as 2500-3000 mg, such as 3000-4000 mg, such as 4000-5000 mg, such as 5000-6000 mg, such as 6000-7000 mg, such as 7000-8000 mg, such as 8000-900 mg, such as 9000 to 10000 mg/day.

In one embodiment said 5-HT1A receptor agonist, or pharmaceutically acceptable derivative thereof, is administered at a daily dosage of 0.01 to 10 mg/kg bodyweight, such as 0.01 to 5 mg/kg bodyweight, such as 0.1 to 1 mg/kg bodyweight.

### Further active ingredients

It is also an aspect of the present disclosure to provide a pharmaceutical composition for use as defined herein, wherein said composition is combined with or comprise one or more further active ingredients which are understood as other therapeutic compounds (active pharmaceutical ingredients) or pharmaceutically acceptable derivatives thereof.

In one embodiment said composition further comprises one or more further active ingredients that are compounds which ameliorate PD symptoms, or which are used for treatment of PD.

These include, but are not limited to, peripheral inhibitors of the transformation of L-DOPA (or other dopamine prodrugs) to dopamine, for example carboxylase inhibitors such as carbidopa (lodosyn) or benserazide; NMDA antagonists such as for example amantadine (Symmetrel, gocrovi, osmolex); catechol-O-methyl transferase (COMT) inhibitors such as for example tolcapone, entacapone, nitecapone and opicapone; MAO-B inhibitors such as for example selegiline and rasagiline; serotonin receptor modulators, kappa opioid receptors agonists, GABA modulators; modulators of neuronal potassium channels such as flupirtine and retigabine; A2A receptor antagonist such as istradefylline, and glutamate receptor modulators such as amantadine, dextromethorphan and deuterated dextromethorphan.

### Examples

**Procedures for evaluating benefits from multiple daily dosages of 5-HT1A agonists in treatment of levodopa induced dyskinesia.**

### General principle

Studies in the rat model of levodopa induced dyskinesia (LID) can be used to determine the effect of 5-HT1A agonists on abnormal involuntary movements (AIMS).

### Simultaneous administration

When a 5-HT1A agonist is given just before (or essentially at the same time as) administration of levodopa a dose dependent reduction in AIMS can be achieved.

When a 5-HT1A agonists is given e.g. 2 hours before levodopa administration, the D/R curve is shifted to the right showing that higher doses of the 5-HT1A agonists is required to reduce AIMS.

A study evaluating adverse effects will show that higher doses of buspirone will cause adverse effects (e.g. sedation), as the literature has shown.

Therefore, it is expected that when the 5-HT1A agonist is given essentially at the same time as levodopa a beneficial efficacy/adverse effect ratio will be obtained.

### Administration with multiple dosages

When a low dose of a 5-HT1A agonist (e.g. 1 mg/kg) is given just before (or essentially at the same time as) administration of levodopa 3 times a day and tested for AIMS every time levodopa is administered (i.e. three times), a reduction in AIMS can be achieved in both each of said AIMS tests and in the total sum of all three AIMS tests.

When a high dose of a 5-HT1A agonist - which dose is the sum of three low dose 5-HT1A as described above (i.e. in this case 3 mg/kg) - is given just before (or essentially at the same time as) administration of levodopa only once in connection with the first dose of levodopa, and levodopa is given alone for the remaining two times, AIMS test conducted after each of said three levodopa administrations can show greater reduction in AIMS at the first AIMS test, but less reduction or no reduction in AIMS at the second and third AIMS test.

Also, the sum of all three AIMS test can show greater reduction in the group administered with low dose 5-HT1A agonists every time levodopa is administered, as compared to the group administered with high dose 5-HT1A agonists only once in connection with the first dose of levodopa (and levodopa given alone for the remaining two times). This can indicate the benefit of frequent dosing of 5-HT1A agonists rather than the dose difference of 5-HT1A agonists.

Therefore, it is expected that when the 5-HT1A agonist is given multiple times with levodopa a beneficial efficacy will be obtained.

**Evaluation of the 5-HT1A agonist buspirone for treatment of movement disorders associated with Parkinson's disease and LID.**

The present study describes the evaluation of the 5-HT1A agonist buspirone in the 6-OHDA rat model.

The 6-OHDA rat model as described below is useful for evaluation of compounds useful for treatment of movement disorders associated with Parkinson's disease and LID. 6-OHDA (6-hydroxydopamine) is a neurotoxin that selectively kills dopaminergic and noradrenergic neurons and induces a reduction of dopamine levels in the brain. Administration of L-DOPA to unilaterally 6-OHDA-lesioned rats induces abnormal involuntary movements (AIMs). These are axial, limb and oral movements that occur only on the body side that is ipsilateral to the lesion. AIM rat models have been shown useful because they respond to a number of drugs which have been shown to suppress dyskinesia (including PD) in humans.

### Test procedure:

Animals: Sprague-Dawley male rats (bred in house, originally from SLAC Laboratory Animal Co. Ltd) at 9-week of age at body weight of 200 to 250 g from Shanghai SLAC Co. Ltd. arrive at the laboratory at least 1 week prior to behavioral testing. Rats are housed in groups of n=2/cage. Animals have ad libitum access to standard rodent chow and water. Animal housing and testing rooms are maintained under controlled environmental conditions and within close proximity of each other. Animal housing rooms are on a 12-hour light-dark cycle with lights on at 6:00 AM and maintained at 70° F/21 °C (range: 68-72° F/20-22°C) with a humidity range of 20-40%. Testing rooms are maintained at 68-72° F with a humidity range of 20-40%.

### 6-OHDA lesion surgery:

Dopamine (DA)-denervating lesions are performed by unilateral injection of 6-OHDA in the ascending nigrostriatal pathway. Rats are anesthetized with pentobarbital sodium 40mg/kg (i.p.) and positioned in a stereotactic frame. 6-OHDA is injected into the right ascending DA bundle at the following coordinates (in mm) relative to bregma and dural surface: (1) toothbar position -2.3, A =-4.4, L = 1.2, V = 7.8, (7.5ug 6-OHDA), (2) toothbar position +3.4, A =-4.0, L = 0.8, V = 8.0mm (6ug 6-OHDA). Alternatively, only one injection is made with the following coordinates: Tooth bar: -3.3mm, AP: -1.8mm, ML: -2.0mm, DV: -8.6mm (18µg/6µl 6-OHDA). The neurotoxin injections are performed at a rate of 1ul/min, and the injection cannula left in place for an additional 2-3 min thereafter.

After recovery from surgery, rats with nearly complete (>90%) lesions are selected by means of an apomorphine-induced rotation test. I.p. injection of 0.5 mg/kg apomorphine·HCl (Sigma) in saline evoked contralateral turning, which is considered to be the result of de-nervated hypersensitivity of DA receptors in the lesion side. Rotational behavior in response to DA agonists grossly correlates with the severity of the lesion. Quantification of the rotational response is accomplished in rats by counting the turns in 30 minutes. Rat with rotational score ≥ 6turns/min are selected for next tests. Animals are then allocated into two well-matched sub-groups (according to the amphetamine rotation) and receive daily treatment as described below.

### Drugs and treatment regimens:

L-DOPA methyl ester (Sigma, Cat No. D9628 Lot. No.030M1604V)) is given at a dose of 6 mg/kg/day, combined with 15 mg/kg/day of benserazide HCl. Chronic treatment with this dose of L-DOPA and benserazide is given for 3 weeks or more to all the rats with good lesions in order to induce a gradual development of dyskinetic-like movements. Thereafter, rats that have not developed dyskinesia are excluded from the study, and the rats with a cumulative AIM score ≥28 points over five testing sessions (dyskinesia severity ≥grade 2 on each axial, limb and orolingual scores) are kept on a drug treatment regimen of at least two injections of L-DOPA/benserazide per week in order to maintain stable AIM scores. The selected rats are allocated groups of 9-12 animals each, which are balanced with respect to AIM severity. The animals are then treated with the drug and drug combinations as described below.

### L-DOPA induced AlMs and drugs screening test:

AlMs ratings are performed by an investigator who is kept unaware of the pharmacological treatment administered to each rat (experimentally blinded). In order to quantify the severity of the AlMs, rats are observed individually in their standard cages every 20th minute at 20-180 min after an injection of I- DOPA. The AIM's are classified into four subtypes:
(A) axial AlMs, i.e., dystonic or choreiform torsion of the trunk and neck towards the side contralateral to the lesion. In the mild cases: lateral flexion of the neck or torsional movements of the upper trunk towards the side contralateral to the lesion. With repeated injection of L-DOPA, this movement may develop into a pronounced and continuous dystonia-like axial torsion.
(B) limb AlMs, i.e., jerky and/or dystonic movements of the forelimb contralateral to the lesion. In mild cases: hyperkinetic, jerky stepping movements of the forelimb contralateral to the lesion, or small circular movements of the forelimb to and from the snout. As the severity of dyskinesia increases (which usually occurs with repeated administration of L-DOPA), the abnormal movements increase in amplitude, and assume mixed dystonic and hyperkinetic features. Dystonic movements are caused by sustained co-contraction of agonist/antagonist muscles; they are slow and force a body segment into unnatural positions. Hyperkinetic movements are fast and irregular in speed and direction. Sometimes the forelimb does not show jerky movements but becomes engaged in a continuous dystonic posture, which is also scored according to the time during which it is expressed.
(C) orolingual AIMs, i.e., twitching of orofacial muscles, and bursts of empty masticatory movements with protrusion of the tongue towards the side contralateral to the lesion. This form of dyskinesia affects facial, tongue, and masticatory muscles. It is recognizable as bursts of empty masticatory movements, accompanied to a variable degree by jaw opening, lateral translocations of the jaw, twitching of facial muscles, and protrusion of the tongue towards the side contralateral to the lesion. At its extreme severity, this subtype of dyskinesia engages all the above muscle groups with notable strength, and may also become complicated by self-mutilative biting on the skin of the forelimb contralateral to the lesion (easily recognizable by the fact that a round spot of skin becomes devoid of fur.
(D) locomotive AIMs, i.e., increased locomotion with contralateral side bias. The latter AIM subtype was recorded in conformity with the original description of the rat AIM scale, although it was later established that locomotive AIMs do not provide a specific measure of dyskinesia, but rather provide a correlate of contralateral turning behavior in rodents with unilateral 6-OHDA lesions.

Each of the four subtypes can be scored on a severity scale from 0 to 4, where 0 = absent, 1 = present during less than half of the observation time, 2 = present for more than half of the observation time, 3 = present all the time but suppressible by external stimuli, and 4 = present all the time and not suppressible by external stimuli.

Rats are tested for AlMs as described above except that the sum of locomotive (LO) or axial (AX), limb (LI), and orolingual (OL) AIM scores per testing session are used for statistical analyses.

### Simultaneous administration

To determine the effects of buspirone given essentially at the same time as levodopa, and buspirone given before levodopa administration, the following group setting was used:
Vehicle: (saline, i.p., 30 min before L-DOPA, n=6)
Buspirone (0.1-10 mg/kg, i.p., n=6) given 180, 120, 90, 60, 30 minutes before L-DOPA.
Buspirone (0.1-10 mg/kg, i.p., n=6) given 5 minutes before L-DOPA and essentially at the same time as L-DOPA.

We have previously shown that buspirone dose dependently reduce L-DOPA-induced involuntary movements.

### Low/high dosage and multiple dosages

To determine the effects of low dose buspirone given multiple times (3 times a day) at the same times as levodopa, and the effects of high dose buspirone given only once at the same time of levodopa administration, the following group setting was used:
Group 1 (n=8):
   Vehicle (saline, i.p., L-DOPA, n=8) given 5-10 minutes before L-DOPA and essentially at the same time as L-DOPA for three times a day
Group 2 (n=8):
   Buspirone (1 mg/kg, i.p., n=8) given 5-10 minutes before L-DOPA and essentially at the same time as L-DOPA for 3 times a day.
Group 3 (n=8):
   Buspirone (3 mg/kg, i.p., n=8) given 5-10 minutes before L-DOPA and essentially at the same time as L-DOPA for only once, at the first dose of L-DOPA.
   Vehicle (saline, i.p., L-DOPA, n=8) given 5-10 minutes before L-DOPA and essentially at the same time as L-DOPA at second and third L-DOPA administration.

AIMS score of each time point, or the sum of all three AIMS test throughout the day between three groups, will be compared to evaluate the efficacy of multiple dosing of buspirone in L-DOPA induced dyskinesia. The total daily amount/dosage of buspirone given is the same in Group 2 and 3, and hence the difference in the sum of AIMS will arise from the frequency of buspirone administrations.

### Evaluation of adverse effects

The present study describes the evaluation of 5-HT1A agonists (e.g. buspirone) in the in models for determination of adverse effects like sedation. Using these methods, a treatment with compounds as disclosed herein can be evaluated for their sedative effects, and/or their effects on the motor performance compared to sedated rats and to rats which only had saline injections.

### Rotarod test

The rotarod test serves the purpose of detecting potential deleterious effects of the compounds studied on the rats' motor performance and coordination. In brief, the animals (30 SD male rats (180-220g, bred in house, originally from SLAC Laboratory Animal Co. Ltd) at 9-week of age) are trained twice a day for a 3-day period. The rats are placed on the accelerating rod apparatus at an initial speed of 4 rotations per minute (rpm), with the speed increasing gradually and automatically to 40 rpm over 300s. Each training trial is ended if the animal falls off or grips the device and spins around for two consecutive revolutions. The time the rat stays on the Rotarod is recorded. The staying duration recorded at last training trail is used as baseline. Rats are grouped according a randomly distribution of baseline.

For the test session on the fourth day, the rats are evaluated on the Rotarod with the same setting as above at 5 and 30 min after dosing. The rats are dosed with drugs as described below. Dosing and Rotarod measurement are conducted by two scientists separately. Pentobarbital (15mg/kg. i.p.) is used a as a positive control.

Group setting for compound tests:
Vehicle: Saline, i.p., 5 or 30 min before test, n=10
Positive control: Pentobarbital 15 mg/kg, i.p. 5 or 30 min before test, n=10
Buspirone (0.1-30 mg/kg, i.p.) 5 or 30min before test, n=10

Statistical analysis: The rotarod performance is expressed as total number of seconds spent on the accelerating rod. The data are analysed using One-Way ANOVA and the Tukey post-hoc test.

### Result:

Buspirone is able to affect performance in the rotarod model in a dose dependent manner compared to rats injected with vehicle only, showing that motor performance and coordination can be significantly reduced in rats after administration of Buspirone. Pentobarbital also significantly reduces time spent on the rotarod.

### Open field test

The open field test is used to determine the effects of drug on locomotor activity. Rats are put in open-field chambers (dimensions 40cm × 40cm × 40cm) 30 minutes after dosing. After a 15 minutes habituation, locomotion is recorded and analysed by Enthovision Video Tracking Software (Noldus Information Technology, Netherlands) for 60 minutes. All locomotor activities are done during dark phase and to eliminate olfactory cues, the arena is thoroughly cleaned with 70%v/v ethanol between each test.

Group setting for compound tests:
Vehicle: Saline, i.p., 5 or 30 min before test, n=10
Positive control: Pentobarbital 15 mg/kg, i.p. 5 or 30 min before test, n=10
Buspirone (0.1-30 mg/kg, i.p.) 5 or 30 min before test, n=10

Statistical analysis: The total locomotor activity is expressed as total moved distance (cm) and average velocity (cm/s) during 60 minutes. The data is analysed using One-Way ANOVA and the Tukey post-hoc test. The locomotor activity in six time point is expressed as moved distance (cm) and average velocity (cm/s) every 10 minutes. The data is analysed using One-Way ANOVA and the Tukey post-hoc test in each time point.

### Result:

Buspirone dose dependently is able to affect performance in the open field test compared to rats injected with vehicle only as measured during the 60 minutes observation period. Pentobarbital significantly reduced motor performance during the total observation period.

Comparing the dose of buspirone that is able to reduce L-DOPA induced abnormal involuntary movements when buspirone is administered at the same time as L-DOPA with the dose of buspirone that induce sedation will determine a therapeutic index. This will illustrate the beneficial therapeutic index in a situation when buspirone is administered together with L-DOPA several times per day.

Comparing the dose of buspirone that is able to reduce L-DOPA induced abnormal involuntary movements when buspirone is administered 120, 90, or 60 min before L-DOPA will illustrate the therapeutic index in a situation when buspirone is administered at different times than L-DOPA 1-2 times per day.

### Revised test procedure for evaluating effects on multiple dosing of buspirone in levodopa induced dyskinesia

### 6-OHDA lesion surgery:

Dopamine (DA)-denervating lesions are performed by unilateral injection of 6-OHDA in the ascending nigrostriatal pathway.

### Apomorphin-induced rotation test:

Screening of the model by subcutaneous injection of apomorphine.

### Drugs and treatment regimens:

L-DOPA methyl ester (Sigma) is given at a dose of 6 mg/kg/day, combined with 15 mg/kg/day of benserazide HCl. Chronic treatment with this dose of L-DOPA and benserazide is given for 3 weeks or more to all the rats with good lesions in order to induce a gradual development of dyskinetic-like movements. Thereafter, rats that have not developed dyskinesia are excluded from the study, and the rats with a cumulative AIM score ≥28 points over five testing sessions (dyskinesia severity ≥ grade 2 on each axial, limb and orolingual scores) are kept on a drug treatment regimen of at least two injections of L-DOPA/benserazide per week in order to maintain stable AIM scores. The selected rats are allocated groups of 6 animals each, which are balanced with respect to AIM severity.

### L-DOPA induced AlMs and drugs screening test:

Buspirone will be dosed at different time points before the L-DOPA dosing as describing above. Rats are observed individually in their standard cages every 20th minute at 20-180 min after an injection of L- DOPA.

### Group setting: N=6, SC:

1. Vehicle: saline, 5-10 min before L-DOPA
2. Buspirone (0.3 mg/kg) given 120 minutes before L-DOPA.
3. Buspirone (1 mg/kg) given 120 minutes before L-DOPA
4. Buspirone (3 mg/kg) given 120 minutes before L-DOPA
5. Buspirone (10 mg/kg) given 120 minutes before L-DOPA
6. Buspirone (0.1 mg/kg) given 5-10 minutes before L-DOPA and essentially at the same time as L-DOPA.
7. Buspirone (0.3 mg/kg) given 5-10 minutes before L-DOPA and essentially at the same time as L-DOPA.
8. Buspirone (1 mg/kg) given 5-10 minutes before L-DOPA and essentially at the same time as L-DOPA.
9. Buspirone (3 mg/kg) given 5-10 minutes before L-DOPA and essentially at the same time as L-DOPA.

## Claims

1. A pharmaceutical composition comprising a 5-HT1A receptor agonist, or a pharmaceutically acceptable salt thereof,
wherein said composition further comprises, separately or together, dopamine, a dopamine agonist, or a dopamine precursor,
wherein said 5-HT1A receptor agonist, or a pharmaceutically acceptable salt thereof, and said dopamine, dopamine agonist, dopamine precursor are to be administered essentially at the same time,
with the proviso that said composition does not comprise an agonist of one or more of the serotonin receptors selected from the group of 5-HT1B, 5-HT1D and/or 5-HT1F receptor, or a pharmaceutically acceptable salt thereof,
wherein said composition is to be administered at least 2 times daily,
for use in the treatment, prevention or alleviation of a movement disorder.

2. The composition for use according to claim 1, wherein said composition is to be administered 2 times daily; such as 3 times daily, such as at least 3 times daily; such as 4 times daily, such as at least 4 times daily; such as 5 times daily, such as at least 5 times daily; such as 6 times daily, such as at least 6 times daily; such as 7 times daily, such as at least 7 times daily; or such as 8 times daily, such as at least 8 times daily.

3. The composition for use according to any of the preceding claims, wherein said composition comprises together i) a 5-HT1A receptor agonist, or a pharmaceutically acceptable salt thereof, and ii) dopamine, a dopamine agonist, or a dopamine precursor, within the same single pharmaceutical composition.

4. The composition for use according to any of the preceding claims, wherein said composition comprises separately i) a 5-HT1A receptor agonist, or a pharmaceutically acceptable salt thereof, and ii) dopamine, a dopamine agonist, or a dopamine precursor, within separate or distinct pharmaceutical compositions, or different compartments of a composite pharmaceutical composition.

5. The composition for use according to any of the preceding claims, wherein said 5-HT1A receptor agonist is selected from the group consisting of buspirone, 6-hydroxybuspirone, tandospirone, gepirone, alnespirone, binospirone, ipsapirone, perospirone, befiradol, repinotan piclozotan, osemozotan, flesinoxan, flibanserin, sarizotan, eltoprazine, F13714 and F15599, or pharmaceutically acceptable salts thereof.

6. The composition for use according to any of the preceding claims, wherein said 5-HT1A receptor agonist is buspirone.

7. The composition for use according to any of the preceding claims, wherein said dopamine precursor is selected from the group consisting of L-phenylalanine, L-tyrosine, L-DOPA, a deuterated analogue of L-DOPA and an ester of L-DOPA.

8. The composition for use according to any of the preceding claims, wherein said dopamine precursor is L-DOPA (levodopa).

9. The composition for use according to any of the preceding claims, wherein said dopamine precursor is
a) a formulation comprising L-DOPA in combination with one or more further active ingredients, such as a DOPA decarboxylase inhibitor and/or a COMT inhibitor, or
b) a formulation comprising L-DOPA in combination with a DOPA decarboxylase inhibitor, such as a DOPA decarboxylase inhibitor selected from the group consisting of carbidopa and benserazide, or
c) a formulation comprising L-DOPA in combination with a COMT inhibitor, such as a COMT inhibitor selected from the group consisting of tolcapone, entacapone, nitecapone and opicapone.

10. The composition for use according to any of the preceding claims, wherein said dopamine agonist is
a) a dopamine receptor agonist, optionally wherein said dopamine receptor agonist is selected from the group consisting of bromocriptine (Parlodel), pergolide (Permax), pramipexole (Mirapex, Sifrol), ropinirole (Requip), piribedil (Pronora, Trivastal), cabergoline (Dostinex), apomorphine (Apokyn), propylnorapomorphine, lisuride, Ciladopa, dihydrexidine, dinapsoline, doxanthrine, epicriptine, quinagolide (Norprolac), rotigotine (Neupro), roxindole, sumanirole and fenoldopam; or
b) an indirect dopamine agonist, such as a dopamine reuptake inhibitor/ transporter blocker and dopamine releasing agent, optionally wherein said indirect dopamine agonist is selected from the group consisting of amphetamine and/or dextroamphetamine, Bupropion (Wellbutrin), Amineptine, nomifensine, Lisdexamfetamine, Methylphenidate (Ritalin) or dexmethylphenidate, methylenedioxypyrovalerone (MDPV; "Sonic"), ketamine, and phencyclidine (PCP), Cathinone, Cocaine, amphetamine, Methamphetamine, methylenedioxymethamphetamine (MDMA), Phenethylamine, p-Tyramine, lisdexamfetamine (Vyvanse), phenmetrazine, pemoline, 4-methylaminorex (4-MAR), and benzylpiperazine, and Vesicular monoamine transporter 2 (VMAT2) inhibitors such as reserpine, tetrabenazine and deserpidine.

11. The composition for use according to any of the preceding claims, wherein said composition comprises or consists of buspirone and levodopa.

12. The composition for use according to any of the preceding claims, wherein
a) said 5-HT1A receptor agonist, or a pharmaceutically acceptable salt thereof, is to be administered simultaneously, separately or sequentially with said dopamine, dopamine agonist or dopamine precursor; or
b) said 5-HT1A receptor agonist, or a pharmaceutically acceptable salt thereof, is to be administered simultaneously, separately or sequentially with said dopamine, dopamine agonist or dopamine precursor for each administration; or
c) said 5-HT1A receptor agonist, or a pharmaceutically acceptable salt thereof, and said dopamine, dopamine agonist or dopamine precursor is to be administered simultaneously for each administration; or
d) said 5-HT1A receptor agonist, or a pharmaceutically acceptable salt thereof, and said dopamine, dopamine agonist or dopamine precursor is to be administered separately or sequentially essentially at the same time for each administration.

13. The composition for use according to any of the preceding claims, wherein
a) the movement disorder is selected from the group of consisting of: a movement disorder associated with altered or impaired synaptic dopamine levels; Parkinson's disease; movement disorders associated with Parkinson's disease such as bradykinesia, akinesia and dyskinesia; tardive dyskinesia and akathisia; or
b) the movement disorder is selected from the group of consisting of: ataxia, dystonia, essential tremor, Huntington's disease, myoclonus, Rett syndrome, Tourette syndrome, Wilson's disease, chorea, Machado-Joseph disease, restless leg syndrome, spasmodic torticollis, and geniospasm or movement disorders associated therewith; or
c) the movement disorder is associated with or caused by treatment of Parkinson's disease, such as antiparkinsonian therapy, including but not limited to dopamine, dopamine agonists, dopamine precursors, dopamine prodrugs, dopamine mimetics and dopaminergic drugs, such as for example L-DOPA; or
d) the movement disorder is L-DOPA induced dyskinesia (LID); or
e) the movement disorder is caused by or associated with drug therapy including neuroleptic drugs, antipsychotics, antidepressants and anti-emetic drugs; or
f) the movement disorder is caused by or associated with withdrawal of drugs including opioids, barbiturates, cocaine, benzodiazepines, alcohol and amphetamine; or
g) the movement disorder is caused by idiopathic disease, genetic dysfunctions, or infection or other conditions which lead to dysfunction of the basal ganglia and/or lead to altered synaptic dopamine levels.

14. The composition for use according to any of the preceding claims, wherein the movement disorder is L-DOPA induced dyskinesia (LID).

## Patentansprüche

1. Pharmazeutische Zusammensetzung, umfassend einen 5-HT1A-Rezeptoragonisten oder ein pharmazeutisch verträgliches Salz davon,
wobei die Zusammensetzung ferner, einzeln oder zusammen, Dopamin, einen Dopaminagonisten oder einen Dopaminvorläufer umfasst,
wobei der 5-HT1A-Rezeptoragonist oder ein pharmazeutisch verträgliches Salz davon und das Dopamin, der Dopaminagonist und der Dopaminvorläufer im Wesentlichen gleichzeitig zu verabreichen sind,
mit der Maßgabe, dass die Zusammensetzung keinen Agonisten eines oder mehrerer der Serotoninrezeptoren, ausgewählt aus der Gruppe 5-HT1B-, 5-HT1D- und/oder 5-HT1F-Rezeptor, oder ein pharmazeutisch verträgliches Salz davon umfasst,
wobei die Zusammensetzung mindestens zweimal täglich zu verabreichen ist,
zur Verwendung bei der Behandlung, Vorbeugung oder Linderung einer Bewegungsstörung.

2. Zusammensetzung zur Verwendung nach Anspruch 1, wobei die Zusammensetzung zweimal täglich zu verabreichen ist; wie etwa 3-mal täglich, wie etwa mindestens 3-mal täglich; wie etwa 4-mal täglich, wie etwa mindestens 4-mal täglich; wie etwa 5-mal täglich, wie etwa mindestens 5-mal täglich; wie etwa 6-mal täglich, wie etwa mindestens 6-mal täglich; wie etwa 7-mal täglich, wie etwa mindestens 7-mal täglich; oder wie etwa 8-mal täglich, wie etwa mindestens 8-mal täglich.

3. Zusammensetzung zur Verwendung nach einem der vorhergehenden Ansprüche, wobei die Zusammensetzung zusammen i) einen 5-HT1A-Rezeptoragonisten oder ein pharmazeutisch verträgliches Salz davon und ii) Dopamin, einen Dopaminagonisten oder einen Dopaminvorläufer in derselben einzelnen pharmazeutischen Zusammensetzung umfasst.

4. Zusammensetzung zur Verwendung nach einem der vorhergehenden Ansprüche, wobei die Zusammensetzung separat i) einen 5-HT1A-Rezeptoragonisten oder ein pharmazeutisch verträgliches Salz davon und ii) Dopamin, einen Dopaminagonisten oder einen Dopaminvorläufer in separaten oder unterschiedlichen pharmazeutischen Zusammensetzungen oder verschiedenen Kompartimenten einer zusammengesetzten pharmazeutischen Zusammensetzung umfasst.

5. Zusammensetzung zur Verwendung nach einem der vorhergehenden Ansprüche, wobei der 5-HT1A-Rezeptoragonist aus der Gruppe bestehend aus Buspiron, 6-Hydroxybuspiron, Tandospiron, Gepiron, Alnespiron, Binospiron, Ipsapiron, Perospiron, Befiradol, Repinotan Piclozotan, Osemozotan, Flesinoxan, Flibanserin, Sarizotan, Eltoprazin, F13714 und F15599 oder pharmazeutisch verträglichen Salzen davon ausgewählt ist.

6. Zusammensetzung zur Verwendung nach einem der vorhergehenden Ansprüche, wobei der 5-HT1A-Rezeptoragonist Buspiron ist.

7. Zusammensetzung zur Verwendung nach einem der vorhergehenden Ansprüche, wobei der Dopamin-Vorläufer aus der Gruppe bestehend aus L-Phenylalanin, L-Tyrosin, L-DOPA, einem deuterierten Analogon von L-DOPA und einem Ester von L-DOPA ausgewählt ist.

8. Zusammensetzung zur Verwendung nach einem der vorhergehenden Ansprüche, wobei der Dopamin-Vorläufer L-DOPA (Levodopa) ist.

9. Zusammensetzung zur Verwendung nach einem der vorhergehenden Ansprüche, wobei der Dopamin-Vorläufer Folgendes ist:
a) eine Formulierung, die L-DOPA in Kombination mit einem oder mehreren weiteren Wirkstoffen, wie etwa einem DOPA-Decarboxylase-Inhibitor und/oder einem COMT-Inhibitor umfasst, oder
b) eine Formulierung, die L-DOPA in Kombination mit einem DOPA-Decarboxylase-Inhibitor umfasst, wie etwa einem DOPA-Decarboxylase-Inhibitor, ausgewählt aus der Gruppe bestehend aus Carbidopa und Benserazid, oder
c) eine Formulierung, die L-DOPA in Kombination mit einem COMT-Inhibitor umfasst, wie etwa einem COMT-Inhibitor, ausgewählt aus der Gruppe bestehend aus Tolcapon, Entacapon, Nitecapon und Opicapon.

10. Zusammensetzung zur Verwendung nach einem der vorhergehenden Ansprüche, wobei der Dopamin-Vorläufer Folgendes ist:
a) ein Dopamin-Rezeptor-Agonist, wobei der Dopamin-Rezeptor-Agonist optional aus der Gruppe bestehend aus Bromocriptin (Parlodel), Pergolid (Permax), Pramipexol (Mirapex, Sifrol), Ropinirol (Requip), Piribedil (Pronora, Trivastal), Cabergolin (Dostinex), Apomorphin (Apokyn), Propylnorapomorphin, Lisurid, Ciladopa, Dihydrexidin, Dinapsolin, Doxanthrin, Epicriptin, Quinagolid (Norprolac), Rotigotin (Neupro), Roxindol, Sumanirol und Fenoldopam ausgewählt ist; oder
b) ein indirekter Dopaminagonist, wie etwa ein Dopaminwiederaufnahmeinhibitor/Transporterblocker und Dopaminfreisetzer, wobei der indirekte Dopaminagonist optional aus der Gruppe bestehend aus Amphetamin und/oder Dextroamphetamin, Bupropion (Wellbutrin), Amineptin, Nomifensin, Lisdexamfetamin, Methylphenidat (Ritalin) oder Dexmethylphenidat, Methylendioxypyrovaleron (MDPV; "Sonic"), Ketamin und Phencyclidin (PCP), Cathinon, Cocain, Amphetamin, Methamphetamin Methylendioxymethamphetamin (MDMA), Phenethylamin, p-Tyramin, Lisdexamfetamin (Vyvanse), Phenmetrazin, Pemolin, 4-Methylaminorex (4-MAR) und Benzylpiperazin sowie Vesicular Monoamine Transporter 2 (VMAT2)-Inhibitoren wie Reserpin, Tetrabenazin und Deserpidin ausgewählt ist.

11. Zusammensetzung zur Verwendung nach einem der vorhergehenden Ansprüche, wobei die Zusammensetzung Buspiron und Levodopa umfasst oder daraus besteht.

12. Zusammensetzung zur Verwendung nach einem der vorhergehenden Ansprüche, wobei
a) der 5-HT1A-Rezeptoragonist oder ein pharmazeutisch verträgliches Salz davon gleichzeitig, getrennt von oder nacheinander mit dem Dopamin, Dopaminagonisten oder Dopaminvorläufer zu verabreichen ist; oder
b) der 5-HT1A-Rezeptoragonist oder ein pharmazeutisch verträgliches Salz davon gleichzeitig, getrennt von oder nacheinander mit dem Dopamin, Dopaminagonisten oder Dopaminvorläufer bei jeder Verabreichung zu verabreichen ist; oder
c) wobei der 5-HT1A-Rezeptoragonist oder ein pharmazeutisch verträgliches Salz davon und das Dopamin, der Dopaminagonist oder der Dopaminvorläufer bei jeder Verabreichung gleichzeitig zu verabreichen sind; oder
d) der 5-HT1A-Rezeptor-Agonist oder ein pharmazeutisch verträgliches Salz davon und das Dopamin, der Dopamin-Agonist oder der Dopamin-Vorläufer bei jeder Verabreichung im Wesentlichen zur gleichen Zeit getrennt oder nacheinander zu verabreichen sind.

13. Zusammensetzung zur Verwendung nach einem der vorhergehenden Ansprüche, wobei
a) die Bewegungsstörung aus der Gruppe bestehend aus Folgendem ausgewählt ist: einer Bewegungsstörung verbunden mit veränderten oder beeinträchtigten synaptischen Dopaminspiegeln; Parkinson-Krankheit; Bewegungsstörungen verbunden mit der Parkinson-Krankheit wie etwa Bradykinesie, Akinesie und Dyskinesie; Spätdyskinesie und Akathisie; oder
b) die Bewegungsstörung aus der Gruppe bestehend aus Folgendem ausgewählt ist: Ataxie, Dystonie, essentiellem Tremor, Huntington-Krankheit, Myoklonus, Rett-Syndrom, Tourette-Syndrom, Wilson-Krankheit, Chorea, Machado-Joseph-Krankheit, Restless-Legs-Syndrom, krampfartiger Torticollis und Geniospasmus oder damit verbundenen Bewegungsstörungen; oder
c) die Bewegungsstörung mit der Behandlung der Parkinson-Krankheit verbunden ist oder durch diese hervorgerufen wird, wie etwa durch eine Anti-Parkinson-Therapie, einschließlich, aber nicht beschränkt auf, Dopamin, Dopaminagonisten, Dopaminvorläufer, Dopamin-Prodrugs, Dopaminmimetika und dopaminerge Arzneimittel, wie etwa L-DOPA; oder
d) die Bewegungsstörung L-DOPA-induzierte Dyskinesie (LID) ist; oder
e) die Bewegungsstörung durch eine medikamentöse Therapie, einschließlich Neuroleptika, Antipsychotika, Antidepressiva und Antiemetika, hervorgerufen wird oder damit verbunden ist; oder
f) die Bewegungsstörung durch den Entzug von Drogen, einschließlich Opioiden, Barbituraten, Kokain, Benzodiazepinen, Alkohol und Amphetamin, hervorgerufen wird oder damit verbunden ist; oder
g) die Bewegungsstörung durch eine idiopathische Erkrankung, genetische Dysfunktionen oder eine Infektion oder andere Zustände hervorgerufen wird, die zu einer Dysfunktion der Basalganglien und/oder zu veränderten synaptischen Dopaminspiegeln führen.

14. Zusammensetzung zur Verwendung nach einem der vorhergehenden Ansprüche, wobei die Bewegungsstörung eine L-DOPA-induzierte Dyskinesie (LID) ist.

## Revendications

1. Composition pharmaceutique comprenant un agoniste du récepteur 5-HT1A, ou un sel pharmaceutiquement acceptable de celui-ci,
dans laquelle ladite composition comprend en outre, séparément ou ensemble, de la dopamine, un agoniste de la dopamine ou un précurseur de la dopamine,
dans laquelle ledit agoniste du récepteur 5-HT1A, ou un sel pharmaceutiquement acceptable de celui-ci, et ladite dopamine, l'agoniste de la dopamine, le précurseur de la dopamine doivent être administrés essentiellement en même temps,
à condition que ladite composition ne comprenne pas d'agoniste d'un ou plusieurs des récepteurs de la sérotonine choisis dans le groupe des récepteurs 5-HT1B, 5-HT1D et/ou 5-HT1F, ou un sel pharmaceutiquement acceptable de celui-ci,
dans laquelle ladite composition doit être administrée au moins 2 fois par jour,
pour une utilisation dans le traitement, la prévention ou le soulagement d'un trouble du mouvement.

2. Composition pour une utilisation selon la revendication 1, dans laquelle ladite composition est à administrer 2 fois par jour ; tel que 3 fois par jour, tel que au moins 3 fois par jour ; tel que 4 fois par jour, tel que au moins 4 fois par jour ; tel que 5 fois par jour, tel que au moins 5 fois par jour ; tel que 6 fois par jour, tel que au moins 6 fois par jour ; tel que 7 fois par jour, tel que au moins 7 fois par jour ; ou tel que 8 fois par jour, tel que au moins 8 fois par jour.

3. Composition pour une utilisation selon l'une quelconque des revendications précédentes, dans laquelle ladite composition comprend de manière combinée, i) un agoniste du récepteur 5-HT1A, ou un sel pharmaceutiquement acceptable de celui-ci, et ii) de la dopamine, un agoniste de la dopamine ou un précurseur de la dopamine, dans la même composition pharmaceutique unique.

4. Composition pour une utilisation selon l'une quelconque des revendications précédentes, dans laquelle ladite composition comprend séparément i) un agoniste du récepteur 5-HT1A, ou un sel pharmaceutiquement acceptable de celui-ci, et ii) de la dopamine, un agoniste de la dopamine ou un précurseur de la dopamine, dans des compositions pharmaceutiques séparées ou distinctes, ou des compartiments différents d'une composition pharmaceutique composite.

5. Composition pour une utilisation selon l'une quelconque des revendications précédentes, dans laquelle ledit agoniste des récepteurs 5-HT1A est choisi dans le groupe constitué de la buspirone, la 6-hydroxybuspirone, la tandospirone, la gépirone, l'alnespirone, la binospirone, l'ipsapirone, la perospirone, le befiradol, le répinotan, le piclozotan, l'osemozotan, le flesinoxan, la flibansérine, le sarizotan, l'éltoprazine, le F13714 et le F15599, ou leurs sels pharmaceutiquement acceptables.

6. Composition pour une utilisation selon l'une quelconque des revendications précédentes, dans laquelle ledit agoniste du récepteur 5-HT1A est la buspirone.

7. Composition pour une utilisation selon l'une quelconque des revendications précédentes, dans laquelle ledit précurseur de dopamine est choisi dans le groupe constitué de la L-phénylalanine, la L-tyrosine, la L-DOPA, un analogue deutérié de la L-DOPA et un ester de la L-DOPA.

8. Composition pour une utilisation selon l'une quelconque des revendications précédentes, dans laquelle ledit précurseur de la dopamine est la L-DOPA (levodopa).

9. Composition pour une utilisation selon l'une quelconque des revendications précédentes, dans laquelle ledit précurseur de la dopamine est
a) une formulation comprenant de la L-DOPA en combinaison avec un ou plusieurs autres ingrédients actifs, tels qu'un inhibiteur de DOPA décarboxylase et/ou un inhibiteur de COMT, ou
b) une formulation comprenant de la L-DOPA en combinaison avec un inhibiteur de la DOPA décarboxylase, tel qu'un inhibiteur de la DOPA décarboxylase choisi dans le groupe constitué de la carbidopa et le bensérazide, ou
c) une formulation comprenant de la L-DOPA en combinaison avec un inhibiteur de COMT, tel qu'un inhibiteur de COMT choisi dans le groupe constitué de la tolcapone, l'entacapone, la nitecapone et l'opicapone.

10. Composition pour une utilisation selon l'une quelconque des revendications précédentes, dans laquelle ledit agoniste de la dopamine est
a) un agoniste des récepteurs de la dopamine, éventuellement dans lequel ledit agoniste des récepteurs de la dopamine est choisi dans le groupe constitué de la bromocriptine (Parlodel), le pergolide (Permax), le pramipexole (Mirapex, Sifrol), le ropinirole (Requip), le piribédil (Pronora, Trivastal), la cabergoline (Dostinex), l'apomorphine (Apokyn), la propylnorapomorphine, la lisuride, le Ciladopa, la dihydrexidine, la dinapsoline, la doxanthrine, l'épiriptine, la quinagolide (Norprolac), la rotigotine (Neupro), le roxindole, le sumanirole et le fénoldopam ; ou
b) un agoniste indirect de la dopamine, tel qu'un inhibiteur de la recapture de la dopamine/un bloqueur de transporteur et un agent libérant de la dopamine, éventuellement dans lequel ledit agoniste indirect de la dopamine est choisi dans le groupe constitué par l'amphétamine et/ou la dextroamphétamine, le bupropion (Wellbutrin), l'amineptine, la nomifensine, la lisdexamfétamine, le Méthylphénidate (Ritalin) ou le dexméthylphénidate, la méthylènedioxypyrovalérone (MDPV ; « Sonic »), la kétamine et la phencyclidine (PCP), la cathinone, la cocaïne, l'amphétamine, la méthamphétamine, la méthylènedioxyméthamphétamine (MDMA), la phénéthylamine, la p-tyramine, la lisdexamfétamine (Vyvanse), la phenmétrazine, la pémoline, le 4-méthylaminorex (4-MAR) et la benzylpipérazine, et les inhibiteurs du transporteur vésiculaire de la monoamine 2 (VMAT2) tels que la réserpine, la tétrabénazine et la déserpidine.

11. Composition pour une utilisation selon l'une quelconque des revendications précédentes, dans laquelle ladite composition comprend ou est constituée de buspirone et de lévodopa.

12. Composition pour une utilisation selon l'une quelconque des revendications précédentes, dans laquelle
a) ledit agoniste du récepteur 5-HT1A, ou un sel pharmaceutiquement acceptable de celui-ci, doit être administré simultanément, séparément ou séquentiellement avec ladite dopamine, agoniste de la dopamine ou précurseur de la dopamine ; ou
b) ledit agoniste du récepteur 5-HT1A, ou un sel pharmaceutiquement acceptable de celui-ci, doit être administré simultanément, séparément ou séquentiellement avec ladite dopamine, agoniste de la dopamine ou précurseur de la dopamine pour chaque administration ; ou
c) dans laquelle ledit agoniste du récepteur 5-HT1A, ou un sel pharmaceutiquement acceptable de celui-ci, et ladite dopamine, l'agoniste de la dopamine, le précurseur de la dopamine doivent être administrés en même temps pour chaque administration ; ou
d) ledit agoniste du récepteur 5-HT1A, ou un sel pharmaceutiquement acceptable de celui-ci, et ladite dopamine, agoniste de la dopamine ou précurseur de la dopamine doivent être administrés séparément ou séquentiellement essentiellement en même temps pour chaque administration.

13. Composition pour une utilisation selon l'une quelconque des revendications précédentes, dans laquelle
a) le trouble du mouvement est choisi dans le groupe constitué de : un trouble du mouvement associé à des niveaux de dopamine synaptique altérés ou altérés ; la maladie de Parkinson ; les troubles du mouvement associés à la maladie de Parkinson tels que la bradykinésie, l'akinésie et la dyskinésie ; la dyskinésie tardive et l'akathisie ; ou
b) un trouble du mouvement choisi dans le groupe constitué de : l'ataxie, la dystonie, le tremblement essentiel, la maladie de Huntington, la myoclonie, le syndrome de Rett, le syndrome de la Tourette, la maladie de Wilson, la chorée, la maladie de Machado-Joseph, le syndrome des jambes sans repos, le torticolis spasmodique et le géniospasme ou des troubles du mouvement associés à ceux-ci ; ou
c) le trouble du mouvement est associé ou causé par le traitement de la maladie de Parkinson, tel qu'un traitement antiparkinsonien, y compris, mais sans s'y limiter, la dopamine, les agonistes de la dopamine, les précurseurs de la dopamine, les prodrogues de la dopamine, les mimétiques de la dopamine et les substances dopaminergiques, comme par exemple la L-DOPA ; ou
d) le trouble du mouvement est la dyskinésie induite par la L-DOPA (LID) ; ou
e) un trouble du mouvement causé par ou associé à une pharmacothérapie comprenant des substances neuroleptiques, des antipsychotiques, des antidépresseurs et des substances antiémétiques ; ou
f) un trouble du mouvement causé par ou associé au sevrage de substances, y compris les opioïdes, les barbituriques, la cocaïne, les benzodiazépines, l'alcool et l'amphétamine ; ou
g) le trouble du mouvement est causé par une maladie idiopathique, des dysfonctionnements génétiques ou une infection ou d'autres conditions qui conduisent à un dysfonctionnement des ganglions de la base et/ou conduisent à des niveaux de dopamine synaptique altérés.

14. Composition pour une utilisation selon l'une quelconque des revendications précédentes, dans laquelle le trouble du mouvement est une dyskinésie L-DOPA-induite (LID).
